# EUROPEAN PATENT SPECIFICATION

(11) **EP 0 601 698 B1**
(45) Date of publication and mention of the grant of the patent: **08.07.1998**
(21) Application number: 93308430.3
(22) Date of filing: 22.10.1993
(51) Int. Cl.: A61K 7/48, A61K 7/06, A61K 35/54

(54) **Topical composition comprising tocopherol**
Topisches Mittel enthaltend Tocopherol
Composition topique contenant du tocopherol

(43) Date of publication of application: 15.06.1994
(73) Proprietor: SHISEIDO COMPANY LIMITED, Chuo-ku, Tokyo 104-8010 (JP)
(72) Inventor: Kakoki, Hiroyuki, c/o Shiseido Research Center, Yokohama-shi, Kanagawa 223 (JP); Kono, Yoshiyuki, c/o Shiseido Research Center, Yokohama-shi, Kanagawa 223 (JP); Funatsu, Shinichiro, c/o Shiseido Research Center, Yokohama-shi, Kanagawa 223 (JP); Komatsu, Masaaki, c/o Shiseido Research Center, Yokohama-shi, Kanagawa 223 (JP)
(74) Representative: Perry, Robert Edward

(56) References cited:
- S.T.N., File Supplier, KARLSRUHE, DE File Chemical Abstracts, vol 116, n 66912 * abstract * & JP-A-03 255 018 (Q.P. CORP.)
- PATENT ABSTRACTS OF JAPAN vol. 16, no. 61 (C-910) & JP-A-03 258 709 (Q.P. CORP.)
- PATENT ABSTRACTS OF JAPAN vol. 13, no. 61 (C-567)(3409) & JP-A-63 254 180 (KOBAYASHI KOOC K.K.)

## Description

This invention relates to a topical composition comprising tocopherol or a tocopherol-type compound.

Lipid peroxide, produced by oxidation of lipid, has various effects on the human body, including cytotoxicity. Humans' natural anti-oxidation function declines in effect with age. In particular, in the skin, which is exposed to external factors, the amount of lipid peroxide increases with age. This is thought to be a cause of skin degradation.

Substances which suppress the formation of lipid peroxide, such as BHA and BHT are known. However, these substances are not satisfactorily safe when applied to the skin long-term.

Vitamins such as ascorbic acid and tocopherol, which can be utilised for cosmetics, are not unsafe. However, owing to coloration when stored, ascorbic acid can be used only in a small amount. It is known that tocopherol can suppress the formation of lipid peroxide in the skin to some degree, but it does not remove existing lipid peroxide.

Chemical Abstracts, vol. 116, no. 66912 (& JP-A-03 255 018), discloses a mixture of a product obtainable by decomposition or hydrolysis (solution) of eggshell membrane and tocopherol or tocopherol derivatives. This mixture exhibits excellent anti-dandruff effects which can also be pathological.

Patent Abstracts of Japan vol. 13, no. 61 (C-567) (3409) (& JP-A-63 254 180), discloses the use of Vitamin E and soluble protein such as soluble collagen, keratin decomposition product, elastin decomposition product, albumin or casein, for suppressing formation of peroxide in the skin.

According to the present invention, it has been found that an improvement in the suppression of lipid peroxide, and removal of the peroxide, can be observed when a compound selected from α-tocopherol, tocopherol acetate, DL-α-tocopherol nicotinate and DL-α-tocopherol succinate, is combined with a product obtained from eggshell membrane. The mixture can be formulated as a topical composition, i.e. a medicament for external use, for application to the skin.

The novel composition comprises an extremely small amount, i.e. 0.00005-0.5 wt%, and more preferably 0.0001-0.05 wt%, of the said product. Higher amounts have little or no increased effect. It is suggested that its effect is as a catalyst, because its suppression of peroxide formation and removal of existing peroxide can be observed at such low levels and for a long period.

The amount of said compound, i.e. which is tocopherol or has its function, is preferably 0.005-5.0 wt%, more preferably 0.01-3.0 wt%, of the composition. Higher amounts have little or no increased effect.

The shell membrane product may be prepared as described in Japanese Patent Laid Open No. 3-258709. In particular, the membrane that adheres to the inside of the shell of the egg of a bird such as a quail is dissolved in a solvent such as water and/or alcohol. The decomposition product is prepared by, for example, solubilising the shell membrane with acid, alkali, an organic solvent, an oxidase or a reductase.

For example, the product is obtained by decomposition in alkaline aqueous organic solvent. Examples of such solvents are mixtures of 70-40% of 0.2-3.0 N aqueous alkali such as sodium hydroxide or potassium hydroxide, and 30-60% of a water-miscible organic solvent such as methanol, ethanol or acetone. The decomposition is preferably conducted for 1-8 hours at 30-60°C. The solution of the soluble shell membrane is obtained by neutralising and filtering the solubilised liquid.

In addition to the essential ingredients described above, a composition of the invention may comprise, as desired or necessary, other ingredients which are used for conventional cosmetics, drugs or quasi-drugs for application to skin. Such ingredients are, for example, vitamin A's such as vitamin A oil, retinol and retinol acetate; vitamin B₂'s such as riboflavin, riboflavin butyrate and flavin adenine dinucleotide; vitamin B₆'s such as pyridoxine hydrochloride and pyridoxine dioctanoate; vitamin C's such as L-ascorbic acid, dipalmitate L-ascorbate, Na L-ascorbate-2-sulfate; pantothenic acids such as calcium pantothenate, D-pantothenyl alcohol, pantothenyl ethyl ether and acetylpantothenyl ethyl ether; vitamin D's such as ergocalciferol and cholecalciferol; nicotinic acids such as nicotinic acid, nicotinic acid amide, benzyl nicotinate; vitamin E's such as α-tocopherol, tocopherol acetate, DL-α-tocopherol nicotinate and DL-α-tocopherol succinate; other vitamins such as vitamin P and biotin; amino acids and derivatives thereof such as glycine, alanine, valine, leucine, isoleucine, serine, threonine, aspartic acid and salts thereof, glutamic acid and salts thereof, lysine, arginine, cysteine, methionine, phenylalanine, tyrosine, histidine, tryptophane, proline, N-acyl acidic amino acid salts such as diethyl-N-palmitoyl L asparaginate and sodium N-coconut oil fatty acid-L-glutamate; acyl neutral amino acid salts such as coconut oil fatty acid-sarcosine triethanol amine and laurolylmethyl-β-alanine sodium; pyrrolidonecarboxylic acid and salts thereof, POE (40) hardened castor oil monopyrrogultamic monoisostearic diester and coconut oil fatty acid-L-ethyl arginate-DL-pyrrolidone-carboxylate; oil contents such as avocado oil, palm oil, peanut oil, beef tallow, rice bran oil, jojoba oil, evening primrose oil, carnauba wax, lanolin, liquid paraffin, squalane, isostearyl palmitate, isostearyl alcohol and glycerin tri-2-ethylhexanate; humectants such as glycerin, sorbitol, polyethylene glycol, 1, 3-butylene glycol, collagen, hyaluronic acid, chondroitin sulfuric acid and sodium dextran sulfate; antioxidants such as sodium erisorbate and parahydroxyanisole; surfactants such as sodium stearyl sulfate, cetyl sulfate diethanol amine, cetyl trimethyl ammonium saccharin, polyethylene glycol isostearate, glyceryl arachate, diglycerin diisostearate and phospholipid; antiseptic agents such as ethyl para-hydroxybenzoate and butyl para-hydroxybenzoate; antiphlogistic agents such as glycyrrhizic acid, glycyrrhetic acid, salicylic acid derivative, hinokitiol, zinc oxide and allantoin; skin beautifiers such as extract of placenta, glutathione and extract of creeping saxifrage; various extracts such as extracts of phellodendron bark, goldthread, peony, Japanese green gentian, birch, sage, loquat, ginseng, aloe, mallow, iris, grape, coix seed, dishcloth gourd, lily, saffron, Cnidium officinare Makino, giner, Saint-John's wort, rosemary and garlic, vitalizers such as royal jelly, sensitizing dye, cholesterol derivatives and extract of calf's blood; blood circulation facilitators such as γ-oryzanol; anti-srborrhoeic agents such as sulfur and thianthol; thickening agents such as carboxyvinyl polymers, carboxymethyl cellulose and carboxylhydroxypropyl cellulose; perfumes; water; alcohols; coloring agents such as titanium yellow, carthamin and safflower red; and resin powder such as polyethylene and nylon powders.

In the present invention, if an ultraviolet absorbent is used in addition to the essential ingredients, the suppressing effect on peroxide is improved.

As the ultraviolet absorbent, ultraviolet absorbents which are permitted as ingredients of ordinary cosmetics are used as occasion demands. Examples thereof are:
cinnamic acid ultraviolet absorbents such as 2-ethoxyethyl paramethoxy cinnamate, isopropyl paramethoxy cinnamate, diisopropyl cinnamate, ethylhexyl paramethoxy cinnamate, glyceryl diparamethoxy cinnamate mono-2-ethyl hexanoate and octyl methoxy cinnamate;
benzoylmethane ultraviolet absorbents such as butylmethoxybenzoylmethane and 4-tert-butyl-4'-methoxy-dibenzoylmethane;
benzophenone ultraviolet absorbents such as glyceryl-mono-2-ethylhexanoyl-di-paramethoxybenzophenone 2-2'-dihydroxy-4-methoxybenzophenone, 2-2'-dihydroxy-4, 4'-dimethoxybenzophenone, 2-hydroxy-4-methoxybenzophenone and sodium 2-hydroxy-4-methoxybenzophenone-5-sulfonate;
benzoic acid ultraviolet absorbents such as methyl orthoaminobenzoate, 2-ethylhexyl-paradimethyl aminobenzoate and octyl paradimethyl aminobenzoate;
benzoate ultraviolet absorbents such as grycelyl paraaminobenzoate, amyl-para-dimethyl aminobenzoate and ethyl-4-bishydroxy propyl aminobenzoate;
   and
other ultraviolet absorbents such as 2-ethylhexyl-2-cyano-3, 3'-diphenyl acrylate, digalloyl trioleate, 2-ethylhexyl salicylate, homomethyl salicylate, guaiazulene and urocanic acid.

The amount of ultraviolet absorbent added is different depending upon the type of ultraviolet absorbent, but it is generally 0.01 to 15.0 wt% of the total amount of external preparation .

If the amount of ultraviolet absorbent is less than 0.01 wt%, the effect is sometimes insufficient. Addition of more than 15.0 wt% of ultraviolet absorbent hardly increases the cutaneous aging resisting effect.

The external preparation according to the present invention may take any given form. For example, it may be a soluble agent such as lotion, an emulsified agent such as milky lotion and cream, an ointment, a dispersant or an aerosol.

In the drawings :
Figure 1 is a diagram illustrating the reinforcement of suppression effect on tocopherol formation of the lipid peroxide by adding the hydrolyzed shell membrane;
Figure 2 is a graph of lipid peroxide formation with time, when tocopherol only, and both of tocopherol and the hydrolyzed shell membrane, are present;
Figure 3 is a diagram illustrating lipid peroxide removing effect by tocopherol, the hydrolyzed shell membrane, and coexistence of both; and,
Figure 4 is a graph of lipid peroxide amount with age, with tocopherol only, the hydrolyzed shell membrane only, or both.

The following Examples illustrate the invention.

First, the preparation example of the soluble shell membrane is explained.

After removing albumen and yolk, an eggshell with the shell membrane was put into water. The eggshell was removed by hand, to obtain a shell membrane. The membrane was dipped in 1% hydrochloric acid aqueous solution for one hour and small pieces of eggshell which adhered to the shell membrane were removed. The shell membrane was washed with water, sun-dried and the dried shell membrane was obtained.

100 g of the dried shell membranes was added into 1200 ml of 2N sodium hydroxide aqueous solution and 800 ml of dehydrated ethanol and stirred for 5 hours at 40°C. In this reaction, the shell membrane was liquidized. After the obtained liquid was filtered with a cloth filter, neutralized and desalted, a 1% aqueous solution of decomposition product was obtained.

Peroxide reaction of purified squalene, in the presence of samples and under UV irradiation, was investigated.

The sample solution (80% of ethanol base) was applied to cover glasses and the solvent was removed (0.2mg/cm² on the cover glasses : approximately corresponding to the level at which a topical preparation is applied to skin). After drying the sample, purified squalene was dropped on it. Following

Ultraviolet irradiation for 1 hour, the squalene was collected with solvent. Amount of the squalene hydroperoxide as the lipid peroxide was measured by CL-HPLC.

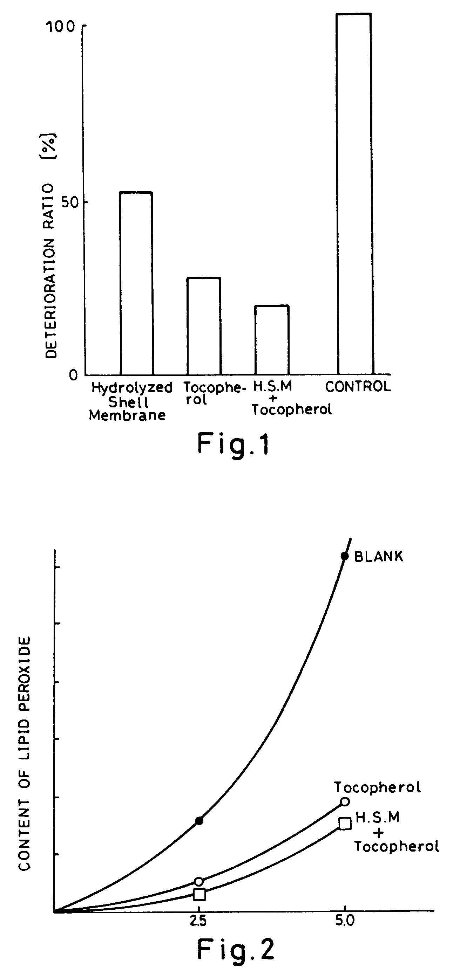

The result is shown in Figure 1.

As is clear from the figure, the lipid peroxide formation was suppressed to about 30% when including 200 ppm α-tocopherol.

In the system including 200 ppm hydrolyzed shell membrane, the suppression effect on lipid peroxide formation was about 50%.

However, when 100 ppm of each substance was used, the lipid peroxide formation was suppressed to about 20%, evidence of a synergistic effect. the hydrolyzed shell membrane and the α-tocopherol.

Further, the present inventors examined the change of the lipid peroxide amount with time under the similar experiment system.

As a result, shown in figure 2, by adding 100 ppm α-tocopherol, the formation curve of lipid peroxide shifts down, and by including also the hydrolyzed shell membrane, the curve shifts further down.

Therefore, it is understood that there is an enhancement of suppression of lipid peroxide formation by adding the hydrolyzed shell membrane to the tocopherol .

In a further test, using a photosensitized oxidation reaction, squalene hydroperoxide was synthesized from purified squalene. Each sample solution (80% of ethanol base) was applied on the cover glasses as described above and dried. A solution of the squalene hydroperoxide was dropped on the cover glasses and the amount of squalene hydroperoxide on them was determined after the 2.5 hours, by the CL-HPLC method.

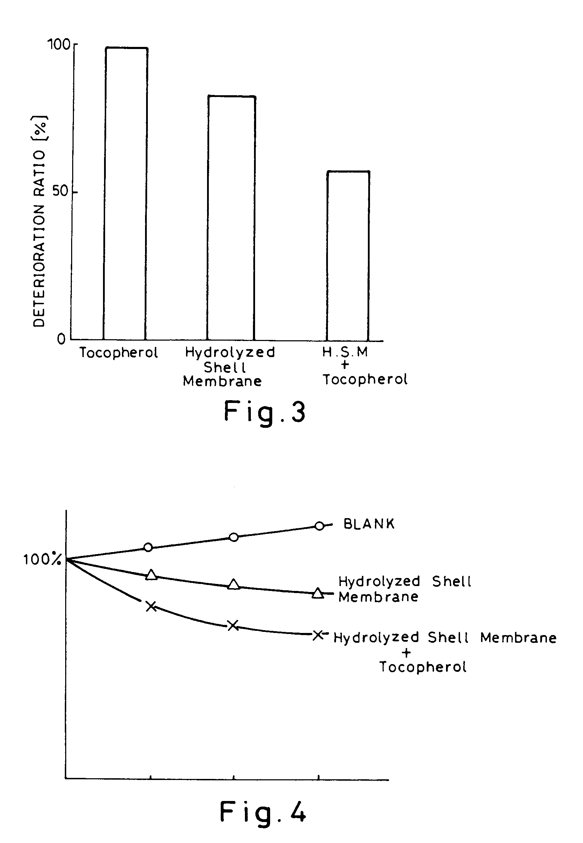

The result is shown in Figure 3.

As is clear from the figure, little effect on removing the lipid peroxide was observed for tocopherol itself. About 20% removal was observed using the hydrolyzed shell membrane. However, when both substances were used, an excellent removing effect on the lipid peroxide (about 40%), was found.

Using a similar experiment system, the change of the lipid peroxide amount with time was observed.

The result is shown in Figure 4.

As is clear from the figure, when using the hydrolyzed shell membrane only, the lipid peroxide decreased with time but reached a stationary state at about 80%.

However, when tocopherol is used as well, the decrease is improved, to about 60%.

From the experimental results, the suppressing effect of tocopherol on lipid peroxide formation is expected by reason of its function as a radical scavenger. However, it is improved synergistically by the hydrolyzed shell membrane. Moreover, while the necessary amount of the hydrolyzed shell membrane is small, the hydrolyzed shell membrane delayed lipid peroxide formation, without being consumed. It is understood that the hydrolyzed shell membrane reinforces the effect of tocopherol like a catalyst.

No lipid peroxide removal is observed for tocopherol, and the effect for the hydrolyzed shell membrane is very weak. Although the hydrolyzed shell membrane has the ability to decompose lipid peroxide, this is catalytic. Therefore, it is not possible to increase peroxide decomposition if the amount of peroxide and that of the decomposed product reach chemical equilibrium. When the hydrolyzed shell membrane and tocopherol are used together, the membrane decomposes hydroperoxide residue in lipid peroxide, by a homolytic reaction. Tocopherol acts as an acceptor of radicals produced on decomposition. As a result, the use of the hydrolyzed shell membrane and tocopherol together have the ability to remove lipid peroxide effectively, in a way that neither can independently.

### Content of Hydrolyzed Shell Membrane

The necessary content of the hydrolyzed shell membrane is examined according to the removing effect on the lipid peroxide.

The result is shown in table 1.

**Table 1**

| | | | | | | | | |
|---|---|---|---|---|---|---|---|---|
| α-tocopherol | 1% | | | | | | | |
| Hydrolyzed shell membrane | 0 | 0.00001 | 0.00005 | 0.0001 | 0.0005 | 0.5 | 1.0 | 3.0 |
| Survival rate of lipid peroxide | 100 | 95 | 70 | 62 | 62 | 60 | 60 | 60 |

As a result, the content of the hydrolyzed shell membrane is equal to or more than 0.00005 wt%. However, addition of more than 0.5 wt% hardly increases the effect.

### Content of Tocopherol

The necessary content of the tocopherol is examined according to the removing effect on the lipid peroxide.

The result is shown in table 2.

**Table 2**

| | | | | | | | | |
|---|---|---|---|---|---|---|---|---|
| α-tocopherol | 0.001 | 0.005 | 0.01 | 0.05 | 0.1 | 0.5 | 1.0 | 5.0 |
| Hydrolyzed Shell membrane | 0.0001 | | | | | | | |
| Survival rate lipid peroxide | 80 | 72 | 60 | 60 | 63 | 60 | 59 | 60 |

As a result, the content of the tocopherol is preferably equal to or more than 0.005 wt%. However, addition of more than 5.0 wt% hardly increases the effect and may reduce the usability of the preparation.

Examples of the external preparation for skin according to the present invention will be explained hereinafter.

### EXAMPLE 1 WATER IN OIL TYPE EMULSIFIED COSMETIC

| 〈Aqueous part〉 | |
|---|---|
| Glycerol | 15.0 weight % |
| 1, 3-butylene glycol | 5.0 |
| Distearyldimethylammomiumhectrite chloride | 2.1 |
| Dimethylpolysiloxane polyethylene glycol | 2.1 |
| 1% Aqueous solution of hydrolyzed shell membrane | 0.01 |
| Ion exchanged water | balance |

| 〈Oil part〉 | |
|---|---|
| Liquid paraffin | 5.0 |
| Vaseline | 1.0 |
| Cetyl 2-ethyl hexanoate | 5.0 |
| Methyl polysiloxane | 5.0 |
| Cyclo polydimethylsiloxane | 10.0 |
| Ethylparaben | 0.2 |
| Butylparaben | 0.1 |
| Tocopherol acetate | 0.07 |

The aqueous part and the oil part were dissolved at 70°C, respectively. The aqueous part was added into the oil part. The system was stirred, emulsified and cooled to room temperature.

### EXAMPLE 2 OIL IN WATER TYPE EMULSIFIED COSMETIC

| 〈Aqueous part〉 | |
|---|---|
| Glycerol | 6.0 weight % |
| 1, 3-butylene glycol | 9.0 |
| Glycerol POE(60) monoisostearate | 1.3 |
| Lipophilic type glyceryl monostearate | 1.6 |
| Self emulsifying propylene glycol monostearate | 1.0 |
| 1% Aqueous solution of hydrolyzed shell membrane | 0.01 |
| Ion exchanged water | Balance |

| 〈Oil part〉 | |
|---|---|
| Cetostearyl alcohol | 3.6 |
| Liquid paraffin | 3.0 |
| Vaseline | 2.5 |
| Cetyl 2-ethyl hexanoate | 3.0 |
| Methyl polysiloxane | 1.5 |
| Ethylparaben | 0.1 |
| Butylparaben | 0.1 |
| Tocopherol acetate | 0.07 |

The aqueous part and the oil part were dissolved at 70°C, respectively. The oil part was added into the aqueous part. The system was stirred, emulsified and cooled to the room temperature.

### Example 3 Lotion

| 〈Aqueous part〉 | |
|---|---|
| 1% Aqueous solution of hydrolyzed shell membrane | 0.05 |
| Sodium 2-hydroxy 4-ethoxybenzophenone 5-sulfonate | 0.1 |
| Glycerin | 4.0 |
| 1,3-butylene glycol | 4.0 |
| Citric acid | 0.05 |
| Sodium citrate | 0.1 |
| Purified water | balance |

| 〈Ethanol part〉 | |
|---|---|
| Tocopherol acetate | 0.01 |
| Ethanol | 8.0 |
| Polyoxyethylene (60) hardened castor oil | 0.5 |
| Methyl para-hydroxybenzoate | 0.2 |
| Perfume | 0.05 |

1% Aqueous solution of hydrolyzed shell membrane, sodium 2-hydroxy-4-methoxybenzophenone-5-sulfonate, citric acid, sodium citrate, glycerin and 1, 3-butylene glycol were dissolved in purified water. Separately from this, polyoxyethylene (60) hardened castor oil, tocopherol acetate, perfume and methyl para-hydroxybenzoate were dissolved in ethanol. The latter solution was added to the purified water solution for solubilization, and the resultant solution was filtered to obtain a lotion.

### Example 4 Cream

| 〈Oil part〉 | |
|---|---|
| Tocopherol acetate | 1.0 |
| Cetostearyl alcohol | 3.5 |
| Squalane | 40.0 |
| Bee wax | 3.0 |
| Reduced lanolin | 5.0 |
| Ethyl para-hydroxybenzoate | 0.3 |
| Polyoxyethylene (20) sorbitan mono palmitate | 2.0 |
| Monoglyceride stearate | 2.0 |
| Sodium N-stearoyl glutamate | 0.5 |
| 2-hydroxy-4-methoxy-benzophenone | 0.5 |
| Octyl methoxycinnamate | 1.0 |
| Retinol acetate | 2.0 |
| Evening primrose oil | 0.05 |
| Perfume | 0.03 |

| 〈Aqueous part〉 | |
|---|---|
| 1% Aqueous solution of hydrolyzed shell membrane | 0.1 |
| 1,3-butylene glycol | 5.0 |
| Polyethylene glycol 1500 | 5.0 |
| Purified water | balance |

Tocopherol acetate, cetostearyl alcohol, squalane, bee wax, reduced lanolin, ethyl para-hydroxybenzoate, polyoxyethylene (20) sorbitan monopalmitate, monoglyceride stearate, sodium N-stearoyl glutamate, 2-hydroxy-4-methoxy-benzophenone, octyl methoxycinnamate, retinol acetate and evening primrose oil were dissolved under heating. Separately from this, 1% aqueous solution of hydrolyzed shell membrane-Na, 1,3-butylene glycol and polyethylene glycol 1500 were heated to 75°C . These liquids were added to purified water under stirring. After pulverizing the emulsified particles by a homomixer, the mixture was rapidly cooled under stirring to produce a cream.

### Example 5 Milky lotion

| 〈Aqueous part〉 | |
|---|---|
| 1% Aqueous solution of hydrolyzed shell membrane | 0.2 |
| L-arginine | 0.3 |
| Na L-glutamate | 0.02 |
| Na hyaluronate | 0.01 |
| Propylene glycol | 5.0 |
| Glycerin | 3.0 |
| Carboxyvinyl polymer | 0.12 |
| PCA-Na | 0.05 |
| Purified water | balance |

| 〈Oil part〉 | |
|---|---|
| 2-ethylhexyl para-dimethylaminobenzoate | 0.1 |
| Mono-2-ethylhexyl diparamethoxycinnamate | 0.2 |
| Stearic acid | 1.5 |
| Cetyl alcohol | 0.5 |
| Bee wax | 2.0 |
| Polyoxyethylene (10) monooleate | 2.0 |
| Ethyl para-hydroxybenzoate | 0.3 |
| Tocopherol acetate | 0.05 |

| 〈Ethanol part〉 | |
|---|---|
| Ethanol | 3.0 |
| Perfume | 0.03 |

Perfume was dissolved in ethanol (alcohol part). 1% Aqueous solution of hydrolyzed shell membrane, L-arginine, Na L-glutamate, PCA-Na, Na hyaluronate, propylene glycol, glycerin and carboxyvinyl polymer were dissolved in purified water under heating and the mixture was held at 70°C (water part). The other ingredients were mixed and dissolved under heating, and the mixture was held at 70 °C (oil part). The oil part was added to the water part for preliminary emulsification and the mixture was uniformly emulsified by a homomixer. The alcohol part was added to the emulsion under stirring. The mixture was cooled to 30 °C under stirring to obtain a milky lotion.

### Example 6 Foam mask

| | |
|---|---|
| 1% Aqueous solution of hydrolyzed shell membrane | 0.02 |
| Glycerin | 5.0 |
| 1,3-butylene glycol | 5.0 |
| Polyethylene glycol 1500 | 3.0 |
| Potassium hydroxide | 0.15 |
| Purified water | balance |

| 〈Oil part〉 | |
|---|---|
| 4-tert-butyl-4'-methoxy-dibenzoylmethane | 0.5 |
| Stearic acid | 1.0 |
| Behenylic acid | 1.0 |
| Self-emulsification type glycerin monostearate | 1.5 |
| Polyoxyethylene monostearate | 2.5 |
| Batyl alcohol | 1.5 |
| Perfume | 0.05 |
| Methyl para-hydroxybenzoate | 0.1 |
| Liquified petroleum gas | 6.0 |
| Dimethyl ether | 2.0 |
| Tocopherol acetate | 0.1 |

1% Aqueous solution of hydrolyzed shell membrane, glycerin, 1,3-butylene glycol, polyethylene glycol 1500, methyl para-hydroxybenzoate and potassium hydroxide were added to purified water and dissolved under heating at 70°C. The other ingredients were added to the mixture with heating and uniformly mixed. The resultant mixture was charged in a container. Finally, liquefied petroleum gas and dimethyl ether were added to the mixture as a spraying agent, thereby producing a foam mask.

### Example 7 Ointment

| 〈Aqueous part〉 | |
|---|---|
| 1% Aqueous solution of hydrolyzed shell membrane | 0.1 |
| Purified water | balance |

| 〈Oil part〉 | |
|---|---|
| Octyl paradimethyl aminobenzoate | 4.0 |
| Butylmethoxybenzoylmethane | 4.0 |
| Tocopherol acetate | 0.5 |
| Retinol palmitate | 1.0 |
| Stearyl alcohol | 18.0 |
| Japan wax | 20.0 |
| Polyoxyethylene (10) monooleate | 0.25 |
| Glycerin monostearate | 0.3 |
| Vaseline | 32.0 |

1% Aqueous solution of hydrolyzed shell membrane was added to purified water and the mixture was held at 70°C (water part). The other ingredients were mixed and dissolved at 70°C (oil part). The oil part was added to the water part and the mixture was uniformly emulsified by a homomixer. The mixture was then cooled to obtain an ointment.

As described above, according to the external preparation of the present invention, since decomposition product of shell membrane and tocopherol and/or derivatives thereof are contained, it is possible to suppress the peroxide formation and further remove the peroxide.

## Claims

1. A topical composition comprising 0.00005 to 0.5 wt% of a product obtainable by decomposition or hydrolysis of eggshell membrane; and a compound selected from α-tocopherol, tocopherol acetate, DL-α-tocopherol nicotinate and DL-α-tocopherol succinate.

2. A composition according to claim 1, wherein said product is obtainable by solubilising the eggshell membrane with acid, alkali, an organic solvent, an oxidase or a reductase.

3. A composition according to claim 1 or claim 2, which comprises 0.005 to 5 wt% of said compound.

4. A cosmetic method for the treatment of the skin, which comprises the topical administration of a composition according to any of claims 1 to 3.

5. Use of a product and a compound as defined in claim 1 or claim 2, for the manufacture of a composition according to any of claims 1 to 3, for use in therapeutic treatment of the skin.

## Patentansprüche

1. Topische Zusammensetzung, umfassend 0,00005 bis 0,5 Gew.-% eines Produktes, das durch Zersetzung oder Hydrolyse von Eierschalen-Membran erhältlich ist; und eine Verbindung, die aus α-Tocopherol, Tocopherolacetat, DL-α-Tocopherolnicotinat und DL-α-Tocopherolsuccinat ausgewählt ist.

2. Zusammensetzung nach Anspruch 1, in welcher das Produkt erhältlich ist durch Solubilisierung der Eierschalen-Membran mit Säure, Alkali, einem organischen Lösungsmittel, einer Oxidase oder einer Reduktase.

3. Zusammensetzung nach Anspruch 1 oder Anspruch 2, welche 0,005 bis 5 Gew.-% der Verbindung umfaßt.

4. Kosmetisches Verfahren zur Behandlung der Haut, umfassend die topische Verabreichung einer Zusammensetzung nach irgendeinem der Ansprüche 1 bis 3.

5. Verwendung eines Produktes und einer Verbindung wie in Anspruch 1 oder 2 definiert für die Herstellung einer Zusammensetzung nach irgendeinem der Ansprüche 1 bis 3 zur Verwendung bei der therapeutischen Behandlung der Haut.

## Revendications

1. Composition topique comprenant de 0,00005 à 0,5 % en poids d'un produit pouvant être obtenu par décomposition ou hydrolyse de membrane coquillère, et un composé choisi parmi l'α-tocophérol, l'acétate de tocophérol, le nicotinate de DL-α-tocophérol et le succinate de DL-α-tocophérol.

2. Composition selon la revendication 1, dans laquelle ledit produit peut être obtenu par dissolution de la membrane coquillère avec un acide, une solution alcaline, un solvant organique, une oxydase ou une réductase.

3. Composition selon la revendication 1 ou 2, comprenant de 0,005 à 5 % en poids dudit composé.

4. Procédé cosmétique pour le traitement de la peau, comprenant l'application locale d'une composition selon l'une quelconque des revendications 1 à 3.

5. Utilisation d'un produit et d'un composé selon la revendication 1 ou 2, pour la préparation d'une composition selon l'une quelconque des revendications 1 à 3, pour utilisation dans le traitement thérapeutique de la peau.
